(19) Europäisches Patentamt
European Patent Office
Office européen des brevets

(11) Veröffentlichungsnummer : **0 097 280**
**B1**

(12) # EUROPÄISCHE PATENTSCHRIFT

(45) Veröffentlichungstag der Patentschrift : **28.08.85**

(51) Int. Cl.$^4$ : **C 07 D249/08**, C 07 D233/60, A 01 N 43/64, A 01 N 43/50

(21) Anmeldenummer : **83105598.3**

(22) Anmeldetag : **08.06.83**

(54) **Azolsubstituierte Oximino-cyan-acetamid-Derivate.**

(30) Priorität : **19.06.82 DE 3222961**

(43) Veröffentlichungstag der Anmeldung : **04.01.84 Patentblatt 84/01**

(45) Bekanntmachung des Hinweises auf die Patenterteilung : **28.08.85 Patentblatt 85/35**

(84) Benannte Vertragsstaaten : **BE CH DE FR GB IT LI NL**

(56) Entgegenhaltungen : **EP-A- 0 005 249**

(73) Patentinhaber : **BAYER AG**
**Konzernverwaltung RP Patentabteilung**
**D-5090 Leverkusen 1 Bayerwerk (DE)**

(72) Erfinder : **Brandes, Wilhelm, Dr.**
**Eichendorffstrasse 3**
**D-5653 Leichlingen 1 (DE)**
Erfinder : **Daum, Werner, Dr.**
**Bärenstrasse 18**
**D-4150 Krefeld 1 (DE)**

**EP 0 097 280 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue azolsubstituierte Oximino-cyan-acetamid-Derivate, mehrere Verfahren zu ihrer Herstellung sowie ihre Verwendung als Fungizide :

Wie bereits lange bekannt ist, werden als Fungizide in der Landwirtschaft und im Gartenbau insbesondere das Zink-ethylen-1,2-bis-dithiocarbamidat und das N-Trichlormethylthio-tetrahydrophthalimid verwendet ; die genannten Verbindungen besitzen unter den Handelsprodukten eine große Bedeutung (vgl. R. Wegler, « Chemie der Pflanzenschutz- und Schädlingsbekämpfungsmittel », Band 2, Seiten 65 und 108, Berlin/Heidelberg/New York (1970)). Die Wirkung bei niedrigen Aufwandkonzentrationen ist jedoch nicht immer befriedigend. Auch sind diese Fungizide nicht kurativ einsetzbar.

Weiterhin aus einer Reihe von Patentschriften bekannt ist die fungizide Wirkung von einigen Isonitroso-cyan-acetamid-Derivaten (vgl. hierzu z. B. die Deutsche Offenlegungsschrift 2 313 956 und die US-Patentschriften 3 919 284, 3 957 847 und 4 188 401). Auch hier ist die Wirksamkeit bei niedrigen Aufwandmengen nicht immer zuverlässig und bei normalen Konzentrationen ist die Pflanzenvertraglichkeit nicht immer voll befriedigend.

Es wurden nun als neue Stoffe die azolsubstituierten Oximino-cyan-acetamid-Derivate der allgemeinen Formel

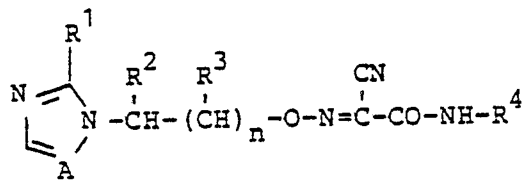

(I)

in welcher

$R^1$ und $R^3$ für Wasserstoff und Alkyl mit bis zu 3 Kohlenstoffatomen stehen,

$R^2$ für Wasserstoff, Alkyl mit bis zu 6 Kohlenstoffatomen und Halogenalkyl mit bis zu 2 Kohlenstoffatomen und bis zu 5 Halogenatomen steht,

$R^4$ für Wasserstoff und die Gruppe CO—NH—$R^5$ steht, wobei

$R^5$ für Wasserstoff, Cycloalkyl mit 3 bis 7 und Alkyl mit bis zu 8 Kohlenstoffatomen steht, wobei letzteres durch Cyano-, Hydroxycarbonyl-, Aminocarbonyl-, Alkoxy-, und Alkoxycarbonyl-Gruppen mit jeweils bis zu 4 Kohlenstoffatomen im Alkylteil substituiert sein kann,

A für N oder die CH-Gruppe steht und

n für die Zahlen 0 oder 1 steht,

gefunden.

Die erfindungsgemäßen Stoffe der allgemeinen Formel (I) können als Oximderivate in 2 verschiedenen geometrischen Strukturen vorliegen :

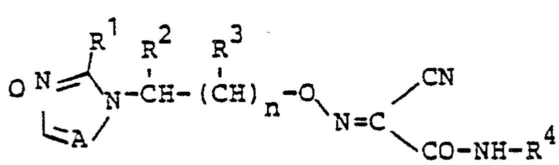

E-Derivat

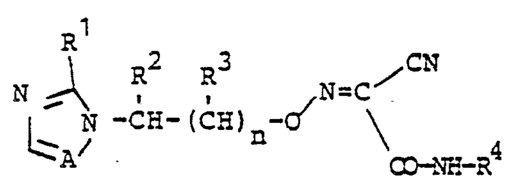

Z-Derivat

Im folgenden wird auf die Angabe der räumlichen Struktur verzichtet ; für die Zwecke der vorliegenden Anmeldung sollen die angegebenen Formeln in jedem Fall auch die entsprechenden Formel gemäß der geometrischen Struktur E oder Z mitumfassen.

Weiterhin wurde gefunden, daß man die azolsubstituierten Oximino-cyan-acetamid-Derivate der Formel (I) erhält, wenn man

a) ein N-Alkylazol der Formel

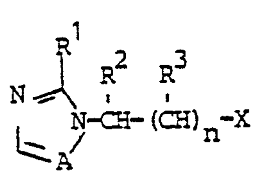

(II)

in welcher

$R^1$, $R^2$, $R^3$, A und n die oben angegebenen Bedeutungen besitzen und

X für eine Abgangsgruppe wie Chlor, Brom, Jod oder eine Sulfonyloxygruppe steht, bzw. dessen Salz (wie z. B. das Hydrohalogenid) mit einem 2-Oximino-2-cyan-acetamid-Derivat der Formel

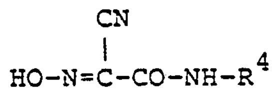

(III)

in welcher
$R^4$ die obengenannte Bedeutung besitzt,
in Gegenwart eines Säurebinders umsetzt ; ferner daß man

b) diejenigen azolsubstituierten Oximino-cyan-acetamid-Derivate der Formel (I), bei denen $R^4$ für die Gruppe CO—NH—$R^5$ steht, wobei $R^5$ für die obengenannten Bedeutungen unter Ausschluß von Wasserstoff und Hydroxycarbonylalkyl steht, auch dann erhält, wenn man gemäß dem Verfahren a) hergestellte erfindungsgemäße Verbindungen der Formel

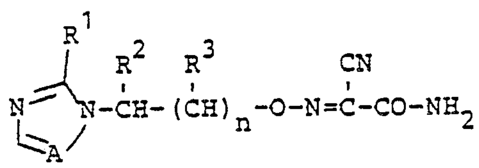

(IV)

in welcher
$R^1$, $R^2$, $R^3$, A und n die obengenannten Bedeutungen besitzen,
(die Formel (IV) ist in dem Fall mit Formel (I) identisch, wenn der Substituent $R^4$ in Formel (I) für Wasserstoff steht),
in Gegenwart einer starken Base mit einem Isocyanat der Formel

$$OCN—R^5 \qquad (V)$$

in welcher
$R^5$ die obengenannten Bedeutungen mit Ausnahme von Wasserstoff und Hydroxycarbonylalkyl besitzt, umsetzt ; und schließlich gemäß einer weiteren Verfahrensvariante

c) Zur Herstellung solcher Verbindungen der Formel (I), bei denen $R^4$ für CO—NH—$R^5$ steht und $R^5$ dabei die Bedeutung Alkyl-carbonyloxyalkyl annimmt, eine nach Verfahren a) synthetisierte Carbonsäure der Formel

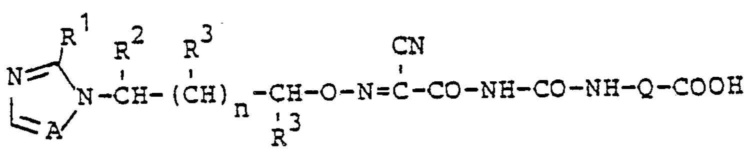

(VI)

in welcher
$R^1$, $R^2$, $R^3$, A und n die obengenannten Bedeutungen besitzen und
Q für gerades oder verzweigtes Alkylen mit 1 bis 8 Kohlenstoffatomen steht
(die Verbindungen der Formel (VI) sind mit den Verbindungen der Formel (I) in dem Falle identisch, wenn der Substituent $R^4$ in Formel (I) für die Gruppe CO—NH—$R^5$ steht und dabei $R^5$ die Bedeutung Hydroxycarbonylalkyl besitzt),
in Form ihres Alkali-, Erdalkali- oder Ammoniumsalzes mit einem Alkylierungsmittel der Formel

$$Y—R^6 \qquad (VII)$$

in welcher
$R^6$ für Alkyl mit 1 bis 4 Kohlenstoffatomen steht und
Y für eine Abgangsgruppe wie Chlor, Brom, Jod, Alkoxysulfonyloxy, Alkylsulfonyloxy oder Arylsulfonyloxy steht,
umsetzt.

Die neuen azolsubstituierten Oximino-cyan-acetamid-Derivate weisen starke fungizide Eigenschaften auf. Sie sind protektiv, kurativ und sogar eradikativ anwendbar, außerdem haben sie systemische und/oder locosystemische Eigenschaften. Überraschenderweise zeigen sie eine bessere Pflanzenverträglichkeit als die nach dem Stand der Technik bekannten Isonitrosocyanacetamid-Derivate. Gegenüber den Dithiocarbamidaten und dem N-Trichlormethylthio-tetrahydrophthalimid besitzen sie den Vorteil curativer und eradikativer Wirkung.

Die erfindungsgemäßen Verbindungen stellen schon wegen der vielen Möglichkeiten ihrer überlegenen biologischen Anwendung eine wertvolle Bereicherung der Technik dar.

Von den erfindungsgemäße, azolsubstituierten Oximino-cyan-acetamid-Derivaten der Formel (I) sind bevorzugt diejenigen, in denen $R^1$ und $R^3$ für Wasserstoff und Methyl stehen, $R^2$ für Wasserstoff und Alkyl mit 1 bis 4 Kohlenstoffatomen steht, $R^4$ für Wasserstoff und die Gruppe CO—NH—$R^5$ steht, wobei $R^5$ für Wasserstoff, für Cycloalkyl mit 3 bis 6 Kohlenstoffatomen und für gegebenenfalls durch Cyano oder Alkoxy-Gruppen mit bis zu 4 Kohlenstoffatomen substituiertes Alkyl mit 1 bis 5 Kohlenstoffatomen steht, und A sowie n die in der Erfindungsdefinition angegebene Bedeutung besitzen.

Besonders bevorzugt sind diejenigen Verbindungen der Formel (I), in denen $R^1$ und $R^3$ für Wasserstoff stehen, $R^2$ für Wasserstoff, Methyl, Ethyl, Isopropyl und sek.-Butyl und $R^4$ für die obengenannten bevorzugten Bedeutungen steht, wobei $R^5$ für Wasserstoff, Methyl, Ethyl, n-Propyl sowie für Isopropyl steht.

Verwendet man zur Herstellung der erfindungsgemäßen Verbindungen der allgemeinen Formel (I) beispielsweise gemäß Verfahrensvariante a) 1-Chlorethyl-triazol-hydrochlorid und 1-(2-Oximino-2-cyan-acetyl)-3-ethyl-harnstoff als Ausgangsstoffe, sowie Ethyl-diisopropylamin als Protonenaktzeptor, so kann der Reaktionsverlauf durch das folgende Formelschema wiedergegeben werden :

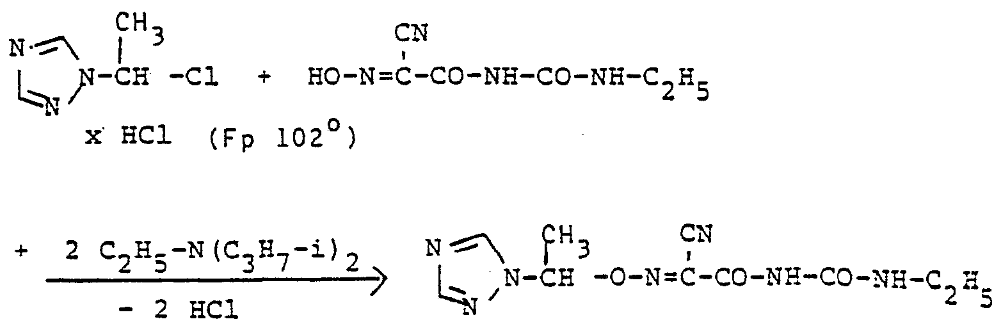


Verwendet man für den Fall, daß in Gegenwart einer anorganischen Base gearbeitet wird, 1-Triazol-1-yl-2-(benzolsulfonyloxy)-propan und das Kaliumsalz des 2-Oximino-2-cyan-acetamids als Ausgangsstoffe, so kann der Reaktionsverlauf durch das folgende Formelschema wiedergegeben werden :

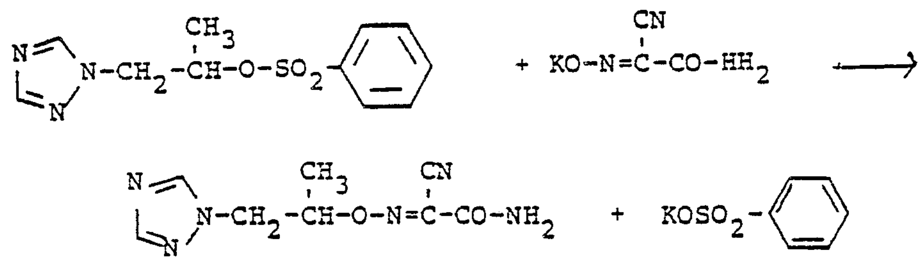

Verwendet man beispielsweise gemäß Verfahrensvariante b) 2-(Triazol-1-yl-methyl-oximino)-2-cyan-acetamid, Natriumhydrid und n-Butylisocyanat als Ausgangsstoffe, so kann der Reaktionsverlauf durch das folgende Formelschema wiedergegeben werden :

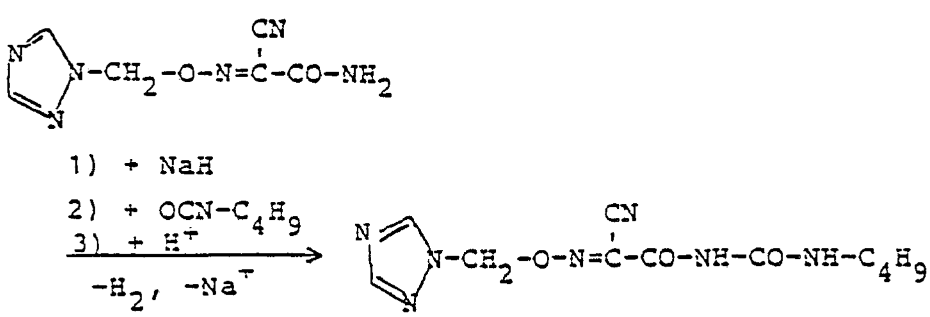


Verwendet man beispielsweise gemäß Verfahrensvariante c) 1-[2-(Triazol-1-yl-methyl-oximino)-2-cyan-acetyl]-3-[5-(hydroxycarbonyl)-pentyl]-harnstoff in Form seines Natriumsalzes und Methyljodid als Ausgangsstoffe, so kann der Reaktionsverlauf durch das folgende Formelschema wiedergegeben werden :

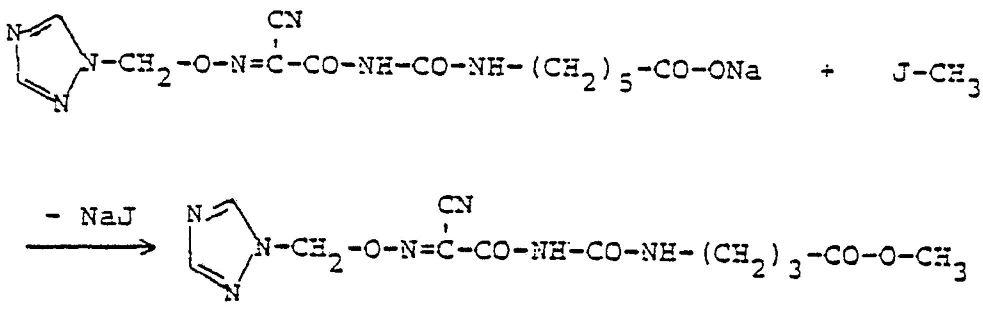


Die nach Verfahrensvariante a) als Ausgangssubstanzen benötigten N-Alkylazole sind durch die Formel (II) definiert. In dieser Formel stehen $R^1$, $R^2$, $R^3$, A und n vorzugsweise für diejenigen Reste, die in Zusammenhang mit den bevorzugten Substituentenbedeutungen für die Formel (I) bereits genannt wurden.

Die Verbindungen der Formel (II) sind teilweise bekannt (Chem. Abstr. *93*, 46530 (1980)). Diejenigen Verbindungen der Formel (II), bei denen der Index n den Wert 0 annimmt, können in enger Analogie zu Literaturangaben (J. Chem. Soc. *1960*, 5272 und DE-OS 2 835 158) z. B. dadurch erhalten werden, daß man einen Aldehyd mit einem Azol, gegebenenfalls in Gegenwart eines Lösungsmittels, zur Reaktion bringt und die erhaltene Methylol-Verbindung anschließend mit Thionylhalogenid umsetzt, wobei dann die N-Alkylazole (II) zumeist als Hydrohalogenide anfallen.

Zu nennen sind hier beispielsweise die folgenden Verbindungen :

1-Chlormethyl-, 1-(1-Chlorethyl)-, 1-(1,2-Dichlorethyl)-, 1-(1-Chlor-2-methyl-propyl)- und 1-(1-Chlorbutyl)-triazol-hydrochlorid, 1-(1-Bromethyl)-triazol-hydrobromid sowie 1-Chlormethyl-, 1-(1-Chlorethyl)-, 1-(1,2-Dichlorethyl)-, 1-(1-Chlor-2-methyl-propyl)- und 1-(1-Chlorpentyl)-imidazol- sowie -2-methylimidazol-hydrochlorid.

Diejenigen Verbindungen der Formel (II), bei denen n für 1 steht, können z. B. aus 2-(Hydroxyalkyl)-azolen durch Umsetzen mit Sulfonsäurechloriden und tertiären Aminen oder Alkalihydriden gewonnen werden.

Zu nennen an Verbindungen der Formel (II), bei denen n für 1 steht, sind zum Beispiel :

2-(Triazol-1-yl)-, 2-(Imidazol-1-yl)-, 2-(2-Methyl-imidazol-1-yl)-ethyl-methansulfonsäureester sowie die entsprechenden Benzolsulfonsäureester, p-Toluolsulfonsäureester und p-Chlorbenzolsulfonsäureester, ferner die Verbindungen 1-(Triazol-1-yl)-2-(benzolsulfonyloxy)-propan und 1-(Imidazol-1-yl)-2-(4-chlorbenzolsulfonyloxy)-butan.

Die weiter oben erwähnten N-(Hydroxyalkyl)-azole werden durch Umsetzen von Azolen mit Alkylenoxiden, wie z. B. Ethylenoxid, in Toluol als Lösungsmittel vorzugsweise bei Raumtemperatur erhalten.

Weiterhin werden für die erfindungsgemäßen Umsetzungen nach Verfahrensvariante a) die 2-Oximino-2-cyan-acetamid-Derivate der allgemeinen Formel (III) benötigt. Die Verbindungen sind teilweise bekannt (vgl. Chem. Ber. *42*, 738-741 (1909) ; *54*, 1334 (1921) ; US-PS 4 188 401). Man erhält sie, wenn man z. B. Ethylisocyanat in einer ersten Stufe mit Ammoniak zu N-Ethyl-harnstoff, in einer zweiten Stufe diesen mit Cyanessigsäure in Gegenwart von Acetanhydrid umsetzt, und den so erhaltenen 1-Ethyl-3-(2-cyanacetyl)-harnstoff mit salpetriger Säure oximiert.

Die für die Verfahrensvariante b) erforderlichen Isocyanate der Formel (V) sind allgemein bekannte Verbindungen ; sie werden in üblicher Weise erhalten, wenn man z. B. primäre Amine mit Phosgen umsetzt.

In Formel (V) hat $R^5$ vorzugsweise diejenigen Bedeutungen, die weiter obei bei Besprechung der Formel (I) vorzugsweise für $R^5$ genannt wurden. An Isocyanaten der Formel (V) sind zu nennen :

Methyl-, Ethyl-, Propyl-, Isopropyl-, Butyl-, sek.-Butyl-, ω-Cyanethyl-, 1-Cyan-1-methyl-ethyl-, ω-Cyanpropyl-, ω-Cyanpentyl-, Methoxycarbonyl-methyl-, Ethoxy-carbonyl-methyl-, Propoxy-carbonyl-ethyl-, 1-Methoxy-carbonyl-1-methyl-ethyl-, 1-Ethoxycarbonyl-1-methyl-ethyl-, Ethoxycarbonyl-1-ethyl-ethyl-, Methoxycarbonyl-1-ethyl-ethyl-, Methoxycarbonyl-propyl-, Methoxycarbonyl-pentyl-, Isopropoxycarbonylpentyl-, Methoxymethyl-, Methoxyethyl-, Cyclopropyl-, Cyclopentyl- und Cyclohexyl-isocyanat.

Als Verdünnungsmittel kommen für das Verfahren gemäß Variante a) alle gegenüber den Reaktionspartnern inerten organischen Lösungsmittel in Frage ; vorzugsweise werden polare Lösungsmittel verwendet. Zu nennen sind hier beispielsweise Acetonitril, Aceton, Chloroform, Benzonitril, Dimethylacetamid, Dimethylformamid, Dimethylsulfoxid, Chlorbenzol, Essigsäureethylester, Dioxan, Methylethylketon, Methylenchlorid und Tetrahydrofuran.

Die Reaktionen können auch in Mischungen aus Wasser und einem wassermischbaren organischen Lösungsmittel durchgeführt werden oder in heterogenen Systemen, bestehend aus Wasser und einem mit Wasser nicht mischbaren oder nur teilweise mischbaren Lösungsmittel.

Als Säurebinder für die Umsetzung nach Verfahren a) werden entweder organische Basen verwendet, vorzugsweise tertiären Amine wie z. B. Chinolin, Dimethylbenzylamin, Dimethylanilin, Ethyldicyclohexylamin, Ethyldiisopropylamin, Piccolin, Pyridin und Triethylamin. Oder aber es wird ein Alkali- oder Erdalkalisalz des 2-Oximino-2-cyan-acetamid-Derivates in einem inerten Lösungsmittel vorgelegt, oder

man erzeugt das Salz, indem man zu einer Mischung aus dem 2-Oximino-2-cyan-acetamid-Derivat und einem hochsiedenden Lösungsmittel Alkalilauge, Alkoholate oder ein Erdalkalihydroxid gibt und dann vorsichtig entwässert bzw. den Alkohol abdestilliert.

Die Reaktionstemperaturen und die Reaktionsdauer beim Verfahren a) werden durch die Aktivität der Ausgangsprodukte bestimmt. Im allgemeinen arbeitet man etwa zwischen − 50 und + 80 °C, vorzugsweise zwischen − 30 und + 40 °C. Bei Verwendung oder Mitverwendung von Wasser als Verdünnungsmittel arbeitet man im Temperaturbereich zwischen dem Erstarrungspunkt der wäßrigen Lösung und etwa + 60 °C, vorzugsweise bei 0 bis + 40 °C.

Zur Durchführung des erfindungsgemäßen Verfahrens nach Variante a) werden das 2-Cyan-2-oximino-acetamid-Derivat der Formel (III) in den erwähnten Verdünnungsmitteln, dispergiert oder gelöst, in stöchiometrischen Mengen vorgelegt und mit einem geringen Überschuß an tertiärem Amin versetzt. Auch kann man das N-Alkylazol (II) in eine Mischung aus Verdünnungsmittel und Alkali-, Erdalkali- oder Ammoniumsalz des 2-Cyan-2-oximino-acetamid-Derivats (III) eintragen. Bei Verwendung eines N-Alkylazol-Salzes wird man entweder vor Zugabe der Azol-Verbindung oder aber gleichzeitig mit dieser nochmals eine molare Menge eines tertiären Amins eintragen.

Die Reaktionsmischung sollte am Ende der Umsetzung alkalisch reagieren, danach aber bald schwach angesäuert werden.

Nach einer besonderen Verfahrensweise setzt man den Mischungen vor Beginn der Reaktion eine kleine Menge eines Jodids zu, wenn man nicht gerade eine Verbindung der Formel (II) mit Jod als Abgangsgruppe einsetzt. Hierdurch wird die Reaktionsgeschwindigkeit erhöht.

Als Verdünnungsmittel kommen für das Verfahren gemäß Variante b) indifferente wasserfreie Lösungsmittel in Frage, wie z. B. Ether, wie Diisopropylether, Dioxan oder Tetrahydrofuran.

Die Reaktionstemperaturen beim Verfahren b) können zwischen − 20 und + 80 °C variiert werden, vorzugsweise arbeitet man zwischen + 20 und 60 °C.

Zur Durchführung des erfindungsgemäßen Verfahrens gemäß Variante b) wird ein 2-Cyan-2-(azol-1-yl-alkyl-oximino)-acetamid-Derivat der Formel (IV) in einem oben näher bezeichneten indifferenten Lösungsmittel mit Natriumhydrid oder Kalium-tert.-butanolat in das entsprechende Alkalisalz überführt und anschließend mit dem Isocyanat der Formel (V) in dem angegebenen Temperaturbereich umgesetzt. Nach beendeter Reaktion wird in der Kälte mit einer organischen Carbonsäure schwach angesäuert.

Als Verdünnungsmittel kommen für das Verfahren gemäß Variante c) alle indifferenten Lösungsmittel in Frage. Vorzugsweise zu nennen sind Acetonitril, Dioxan, oder Ketone, wie z. B. Aceton und Diethylketon.

Die Reaktionstemperaturen beim Verfahren c) können zwischen − 20 und + 80 °C variiert werden, vorzugsweise arbeitet man zwischen + 20 und 60 °C.

Nach Verfahren c) wird man das nach Verfahren a) gewonnene 1-(2-Cyan-2-(azol-1-yl-alkyl-oximino))-3-(ω-carboxyalkyl)-harnstoff-Derivat der Formel (VI) in den angegebenen Lösungsmitteln, nach erfolgter Salzbildung mit einem Alkali- oder Erdalkali-hydroxid oder -carbonat bzw. mit einem tert. Amin, mit einem Alkylierungsmittel der Formel (VII) umsetzen.

Je nach Arbeitsbedingungen fallen die erfindungsgemäßen Wirkstoffe kristallin aus oder sie bleiben im organischen Lösungsmittel gelöst und können dann nach Auswaschen der Lösung mit Wasser durch vorsichtiges Einengen der Lösung oder durch Zugabe wenig polarer organischer Lösungsmittel, wie Cyclohexan, Dibutylether oder Tetrachlorkohlenstoff, abgeschieden werden. Gegebenenfalls müssen mit Wasser mischbare polare Lösungsmittel nach der Reaktion durch Abdampfen im Vakuum entfernt werden.

Sind die erfindungsgemäßen Verbindungen in einem mit Wasser mischbaren Lösungsmittel gelöst, so können sie gegebenenfalls auch durch Zugabe von Wasser ausgefällt werden. Soweit es die besonderen Bedingungen der Aufarbeitungsprozesse erlauben, werden die Lösungen der erfindungsgemäßen Wirkstoffe, bzw. die noch Lösungsmittelfeuchten Suspensionen der Wirkstoffe schwach sauer eingestellt.

Einige Verbindungen sind auch gut wasserlöslich.

Die erfindungsgemäßen Verbindungen zersetzen sich zum Teil bei höherer Temperatur : in diesen Fällen können die Schmelzpunkte nur mit geringer Genauigkeit oder überhaupt nicht ermittelt werden. Das Vorliegen bestimmter Strukturelemente ist aus den NMR-Spreken zu entnehmen. Auch zeigen die IR-Spektren charakteristische Absorptionsbanden.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Oomyceten, z. B. gegen den Erreger der Kraut- und Braunfäule der Kartoffel und Tomate (phytophthora infestans), eingesetzt werden.

Sie zeigen eine hohe kurative und protektive Wirksamkeit. Daneben sind gute Wirkungen gegen Rostpilze festzustellen.

Die erfindungsgemäßen Wirkstoffe weisen nicht nur die guten Eigenschaften hervorragender Handelspräparate auf, sondern besitzen darüber hinaus noch erhebliche Vorteile. Diese liegen in erster Linie in der Fähigkeit der erfindungsgemäßen Stoffe, in die Pflanze einzudringen. Sie können aufgenommen werden von der Saatgutoberfläche, von den Wurzeln und auch von oberirdischen Pflanzenorganen nach äußerlichen Applikationen. Auch besitzen sie die vorteilhafte Fähigkeit, locosystemisch zur Wirkung zu kommen, d. h. eine Tiefenwirkung im Pflanzengewebe auszuüben und dabei pilzliche Krankheitserreger zu elminieren, die bereits in das Gewebe der Wirtspflanze eingedrungen sind.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in Polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z. B. nichtionogene und anionische Emulgatoren, wie Polyoxyethylen-Fettsäure-Ester, Polyoxyethylen-Fettalkohol-Ether, z. B. Alkylarylpolyglykol-ether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Zusätzlich zu den obigen Formulierungsmöglichkeiten ist zu bemerken, daß die erfindungsgemäßen Stoffe zusammen mit Saccharose, Dextrose, Dextrinen, mit wasserfreiem Calciumsulfat oder Calciumsulfat-hemihydrat, sowie mit Carbonsäuren, wie z. B. Fumarsäure oder 4-Hydroxybenzoesäure, oder auch mit schwach sauren Ionenaustauschern formuliert werden können.

Es können in den Formulierungen Haftmittel wie Carboxymethylcellulose, natürliche und synthetische pulverige, körnige und latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanznährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchtsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 %, am Wirkungsort erforderlich.

## Herstellungsbeispiele

### Beispiel 1

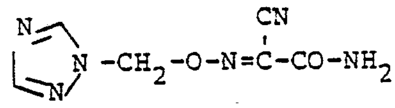

(Verfahren a)

61,1 g (1 Mol) 1,2,4-Triazol und 30 g (1 Mol) Paraformaldehyd werden in 700 ml alkoholfreiem Chloroform während 4 Stunden am Rückfluß gekocht. Die erhaltene Lösung wird bei Raumtemperatur filtriert und das Filtrat unter Kühlen mit einer Eis/Kochsalz-Kältemischung im Verlauf von 2 Stunden zu 250 g (2,1 Mol) Thionylchlorid getropft. Man läßt 17 Stunden stehen und dampft dann im Vakuum ein. Der Rückstand, 1-(Chlor-methyl)-triazol-hydrochlorid, kristallisiert beim Rühren mit 800 ml Acetonitril aus. Er wird sofort weiterverarbeitet.

Man fügt 113 g (1 Mol) 2-Cyan-2-oximinoacetamid zu der oben erhaltenen Mischung hinzu und tropft bei − 2 °C Triethylamin ein, bis eine Probe der Reaktionsmischung mit Wasser einen pH-Wert von 9 anzeigt. Man bringt den pH-Wert durch Zugabe von Essigsäure auf etwa 5 bis 7. Der Niederschlag von Triethylaminhydrochlorid wird abgetrennt, die Lösung in Vakuum eingedampft und der Rückstand zur weiteren Abscheidung vom Triethylamin-Hydrochlorid mit Diethylketon behandelt. Die Diethylketon-Lösung wird im Vakuum eingedampft und der Rückstand durch Zugabe von Butylalkohol zur Kristallisation gebracht. Man erhält 68,7 g 2-Cyan-2-(triazol-1-yl-methoximino)-acetamid vom Fp. 156,5 °C, das sind 36 % der Theorie (unter Einschluß der Vorstufe).

### Beispiel 2

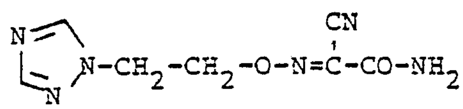

(Verfahren a)

15,1 g (0,1 Mol) Kaliumsalz des 2-Cyan-2-oximinoacetamids und 28,3 g (0,106 Mol) 1-(2-(p-Toluolsulfonyloxy)-ethyl)-triazol werden in 100 ml Acetonitril während 10 Stunden auf 80 °C erhitzt. Das Festprodukt wird abgetrennt und die Acetonitril-Lösung im Vakuum eingedampft. Der Rückstand wird aus siedendem Diethylketon umkristallisiert. Man erhält 13,4 g 2-Cyan-2-[2-(triazol-1-yl)-ethyloximino]-acetamid vom Fp. 137 °C, das sind 64 % der Theorie.

Vorprodukt

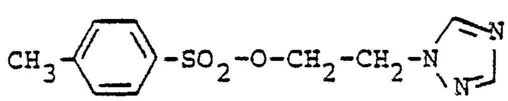

6,4 g einer 75 bis 80 %igen Zubereitung von Natriumhydrid und 300 ml Tetrahydrofuran werden bei 51 bis 54 °C vorgelegt und 22,6 g 1-(2-Hydroxyethyl)-triazol im Verlaufe von 30 Minuten eingetragen. Man beläßt noch weitere 90 Minuten auf dieser Temperatur. Anschließend werden bei − 5 °C 38 g p-Toluolsulfonsäure-chlorid eingetragen. Nach beendeter Reaktion wird im Vakuum eingedampft und der Rückstand mit Wasser versetzt. 1-(2-(p-Toluolsulfonyloxy)-ethyl)-triazol kann nach dem Trocknen mit Petrolether angerieben werden. Die Ausbeute beträgt 34 g, der Schmelzpunkt liegt bei 84 °C.

In entsprechender Weise werden die folgenden Verbindungen der allgemeinen Formel (I) erhalten :

Beispiel 3 : 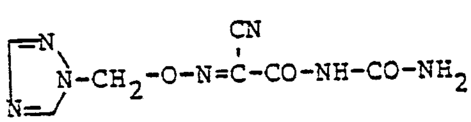 Fp. 185-194 °C

Beispiel 4 : 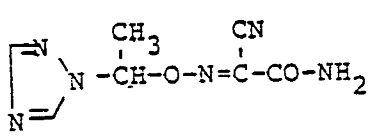 Fp. 130 °C

Beispiel 5 : 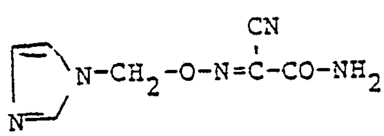 Fp. 179 °C

Beispiel 6 : 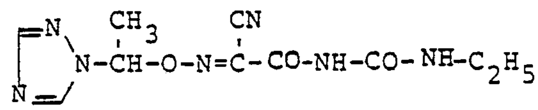 Fp. 120,5 °C

Beispiel 7 : 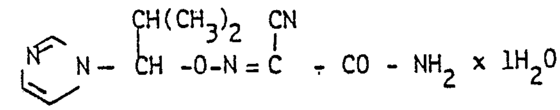 hygroskopisch
$H^1$—NMR N—CH—O
d∫ = 5,83 ppm

Beispiel 8 : 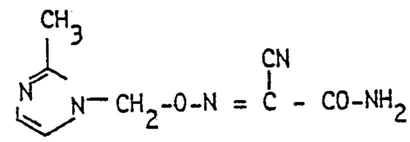 Fp 160°

Beispiel 9 : 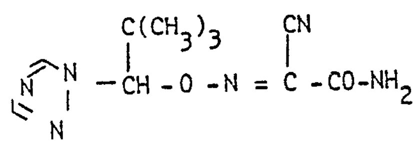 $H^1$—NMR N—CH—O
s∫ = 6,03 ppm
viskoses Produkt

Einzelne Vorprodukte der allgemeinen Formel (II) :

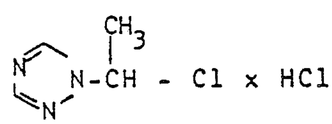 Fp (93-) 102°

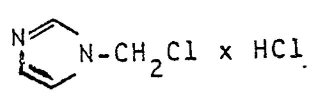 äußerst hygroskopisch

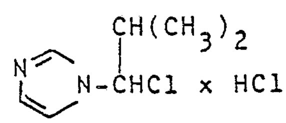 Fp (138-) 154°

Beispiel A

Phytophthora-Test (Tomate) / kurativ

Lösungsmittel : 4,7 Gewichtsteile Aceton
Emulgator : 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Sporensuspension von Phytophthora infestans inokuliert. Die Pflanzen verbleiben 7 Stunden bei 20 °C und 100 % relativer Luftfeuchtigkeit in einer Inkubationskabine. Nach einer kurzen Abtrocknungszeit werden die Pflanzen mit der Wirkstoffzubereitung tropfnaß gespritzt.

Die Pflanzen werden in einer Inkubationskabine mit 100 % relativer Luftfeuchtigkeit und ca. 20 °C aufgestellt.

3 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele : 1 und 2.

**Patentansprüche**

1. Azol-substituierte Oximino-cyan-acetamid-Derivate der allgemeinen Formel

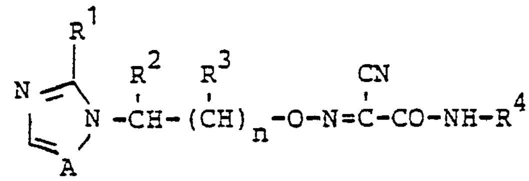 (I)

in welcher

$R^1$ und $R^3$ für Wasserstoff und Alkyl mit bis zu 3 Kohlenstoffatomen stehen,

$R^2$ für Wasserstoff, Alkyl mit bis zu 6 Kohlenstoffatomen und Halogenalkyl mit bis zu 2 Kohlenstoffatomen und bis zu 5 Halogenatomen steht,

$R^4$ für Wasserstoff und die Gruppe CO—NH—$R^5$ steht, wobei

$R^5$ für Wasserstoff, Cycloalkyl mit 3 bis 7 und Alkyl mit bis zu 8 Kohlenstoffatomen steht, wobei letzteres durch Cyano-, Hydroxycarbonyl-, Aminocarbonyl-, Alkoxy- und Alkoxycarbonyl-Gruppen mit jeweils bis zu 4 Kohlenstoffatomen im Alkylteil substituiert sein kann,

A für N oder die CH-Gruppe steht und

n für die Zahlen 0 oder 1 steht.

2. Verbindungen der allgemeinen Formel (I) in Anspruch 1, wobei

A für ein Stickstoffatom oder die CH-Gruppe steht,

$R^1$ und $R^3$ für Wasserstoff oder Methyl stehen ;

$R^2$ für Wasserstoff oder Alkyl mit 1 bis 4 Kohlenstoffatomen steht ;

$R^4$ für Wasserstoff oder die Gruppe CO—NH—$R^5$ steht, wobei

$R^5$ für Wasserstoff, für Cycloalkyl mit 3 bis 6 Kohlenstoffatomen oder für gegebenenfalls durch Cyano oder Alkoxy-Gruppen mit bis zu Kohlenstoffatomen substituiertes Alkyl mit 1 bis 5 Kohlenstoffatomen im Alkylteil steht und

n für die Zahlen 0 oder 1 steht.

3. Verfahren zur Herstellung von azolsubstituierten Oximino-cyan-acetamid-Derivaten der allgemeinen Formel

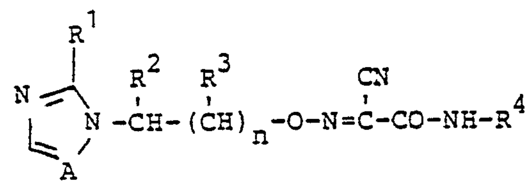 (I)

in welcher

$R^1$ und $R^3$ für Wasserstoff und Alkyl mit bis zu 3 Kohlenstoffatomen steht,

$R^2$ für Wasserstoff, Alkyl mit bis zu 6 Kohlenstoffatomen und Halogenalkyl mit bis zu 2 Kohlenstoffatomen und bis zu 5 Halogenatomen steht,

$R^4$ für Wasserstoff und die Gruppe CO—NH—$R^5$ steht, wobei

$R^5$ für Wasserstoff, Cycloalkyl mit 3 bis 7 und Alkyl mit bis zu 8 Kohlenstoffatomen steht, wobei letzteres durch Cyano-, Hydroxycarbonyl-, Aminocarbonyl-, Alkoxy- und Alkoxycarbonyl-Gruppen mit jeweils bis zu 4 Kohlenstoffatomen im Alkylteil substituiert sein kann,

A N oder CH bedeutet und

n für die Zahlen 0 oder 1 steht,

dadurch gekennzeichnet, daß man

a) ein N-Alkylazol der Formel

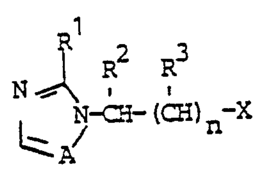 (II)

in welcher

$R^1$, $R^2$, $R^3$, A und n die oben angebene Bedeutungen besitzen und

X für eine Abgangsgruppe wie Chlor, Brom, Jod oder eine Sulfonyloxygruppe steht,

bzw. dessen Salz mit einem 2-Oximino-2-cyan-acetamid-Derivat der Formel

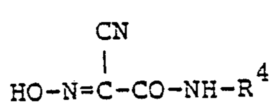 (III)

in welcher

$R^4$ die obengenannte Bedeutung besitzt,

in Gegenwart eines Säurebinders umsetzt ; ferner daß man

b) diejenigen azolsubstituierten Oximino-cyan-acetamid-Derivate der Formel (I), bei denen $R^4$ für die Gruppe CO—NH—$R^5$ steht, wobei $R^5$ für die obengenannten Bedeutungen unter Ausschluß von Wasserstoff und Hydroxycarbonylalkyl steht, auch dann erhält, wenn man gemäß dem Verfahren a) hergestellte erfindungsgemäße Verbindungen der Formel

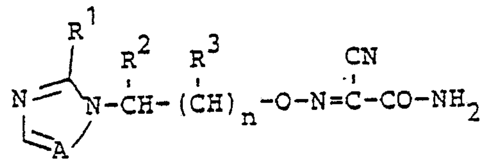

(IV)

in welcher

$R^1$, $R^2$, $R^3$, A und n die obengenannten Bedeutungen besitzen,

in Gegenwart einer starken Base mit einem Isocyanat der Formel

$$OCN—R^5 \qquad (V)$$

in welcher

$R^5$ die obengenannten Bedeutungen mit Ausnahme von Wasserstoff und Hydroxycarbonylalkyl besitzt,

umsetzt ; und daß man

c) zur Herstellung solcher Verbindungen der Formel (I), bei denen $R^4$ für CO—NH—$R^5$ steht und $R^5$ dabei die Bedeutung Alkyl-carbonyloxyalkyl annimmt, eine nach Verfahren a) synthetisierte Carbonsäure der Formel

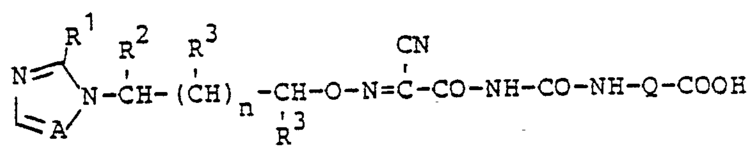

(VI)

in welcher

$R^1$, $R^2$, $R^3$, A und n die obengenannten Bedeutungen besitzen,

Q für gerades oder verzweigtes Alkylen mit 1 bis 8 Kohlenstoffatomen steht,

in Form ihres Alkali-, Erdalkali- oder Ammoniumsalzes mit einem Alkylierungsmittel der Formel

$$Y—R^6 \qquad (VII)$$

in welcher

$R^6$ für Alkyl mit 1 bis 4 Kohlenstoffatomen steht und

Y für eine Abgangsgruppe wie Chlor, Brom, Jod, Alkoxysulfonyloxy, Alkylsulfonyloxy oder Arylsulfonyloxy steht,

umsetzt.

4. Fungizide Mittel, gekennzeichnet durch einen Gehalt an mindestens einem azolsubstituierten Oximino-cyan-acetamid-Derivat der allgemeinen Formel (I) gemäß Ansprüchen 1 und 3.

5. Verfahren zur Bekämpfung von Pilzen, dadurch gekennzeichnet, daß man azolsubstituierte Oximino-cyan-acetamid-Derivate der Formel (I) gemäß Ansprüchen 1 und 3 auf Pflanzen oder ihren Lebensraum einwirken läßt.

6. Verwendung von azolsubstituierten Oximino-cyan-acetamid-Derivaten der Formel (I) gemäß Ansprüchen 1 und 3 zur Bekämpfung von Pilzen.

7. Verfahren zur Herstellung von fungiziden Mitteln, dadurch gekennzeichnet, daß man azolsubstituierte Oximino-cyan-acetamid-Derivate der Formel (I) gemäß Ansprüchen 1 und 3 mit Streckmitteln und/oder oberflächenaktiven Mitteln vermischt.

**Claims**

1. Azole-substituted oximino-cyano-acetamide derivatives of the general formula

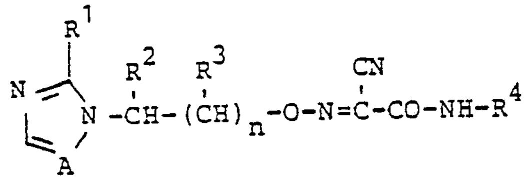

(I)

in which

$R^1$ and $R^3$ represent hdyrogen and alkyl having up to 3 carbon atoms,

$R^2$ represents hydrogen, alkyl having up to 6 carbon atoms and halogenoalkyl having up to 2 carbon atoms and up to 5 halogen atoms,

$R^4$ represents hydrogen and the group CO—NH—$R^5$, wherein

$R^5$ represents hydrogen, cycloalkyl having 3 to 7 and alkyl having up to 8 carbon atoms, it being possible for the latter to be substituted by cyano, hydroxycarbonyl, aminocarbonyl, alkoxy and alkoxycarbonyl groups, each having up to 4 carbon atoms in the alkyl part,

A represents N or the CH group and

n represents the numbers 0 or 1.

2. Compounds of the general formula (I) in Claim 1, wherein

A represents a nitrogen atom or the CH group,

$R^1$ and $R^3$ represent hydrogen or methyl,

$R^2$ represents hydrogen or alkyl having 1 to 4 carbon atoms,

$R^4$ represents hydrogen or the CO—NH—$R^5$ group, wherein

$R^5$ represents hydrogen, cycloalkyl having 3 to 6 carbon atoms or alkyl which has 1 to 5 carbon atoms in the alkyl part and is optionally substituted by cyano or alkoxy groups having up to 4 carbon atoms, and

n represents the numbers 0 or 1.

3. Process for the preparation of azole-substituted oximino-cyano-acetamide derivatives of the general formula

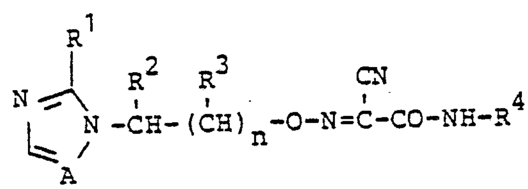

(I)

in which

$R^1$ and $R^3$ represent hydrogen and alkyl having up to 3 carbon atoms,

$R^2$ represents hydrogen, alkyl having up to 6 carbon atoms and halogenoalkyl having up to 2 carbon atoms and up to 5 halogen atoms,

$R^4$ represents hydrogen and the group CO—NH—$R^5$, wherein

$R^5$ represents hydrogen, cycloalkyl having 3 to 7 and alkyl having up to 8 carbon atoms, it being possible for the latter to be substituted by cyano, hydroxycarbonyl, aminocarbonyl, alkoxy and alkoxycarbonyl groups, each having up to 4 carbon atoms in the alkyl part,

A represents N or CH and

n represents the numbers 0 or 1,

characterised in that

a) an N-alkylazole of the formula

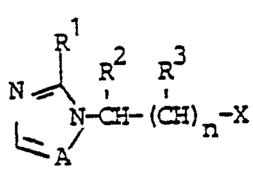

(II)

in which

$R^1$, $R^2$, $R^3$, A and n have the meanings given above and

X represents a leaving group, such as chlorine, bromine, iodine or a sulphonyloxy group,

or its salt, is reacted with a 2-oximino-2-cyano-acetamide derivative of the formula

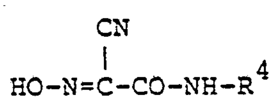

(III)

in which

$R^4$ has the abovementioned meaning,

in the presence of an acid-binding agent ; or that

b) those azole-substituted oximino-cyano-acetamide derivatives of the formula (I), in which $R^4$ represents the CO—NH—$R^5$ group, wherein $R^5$ has the abovementioned meanings, with the exception of hydrogen and hydroxycarbonylalkyl, are also obtained when compounds according to the invention, prepared according to process a) and of the formula

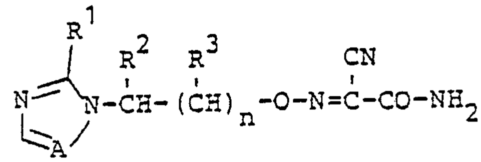

(IV)

in which

$R^1$, $R^2$, $R^3$, A and n have the abovementioned meanings,

are reacted with an isocyanate of the formula

$$OCN—R^5 \qquad (V)$$

in which

$R^5$ has the abovementioned meanings, with the exception of hydrogen and hydroxycarbonylalkyl, in the presence of a strong base ; and that

c) for the preparation of those compounds of the formula (I) in which $R^4$ represents CO—NH—$R^5$ and $R^5$ has the meaning of alkyl-carbonyloxyalkyl in this formula, a carboxylic acid synthesised by process a) of the formula

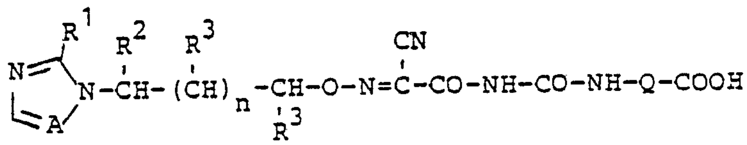

(VI)

in which

$R^1$, $R^2$, $R^3$, A and n have the abovementioned meanings and

Q represents straight or branched alkylene having 1 to 8 carbon atoms,

in the form of its alkali metal, alkaline earth metal or ammonium salt, is reacted with an alkylating agent of the formula

$$Y—R^6 \qquad (VII)$$

in which

$R^6$ represents alkyl having 1 to 4 carbon atoms and

Y represents a leaving group, such as chlorine, bromine, iodine, alkoxysulphonyloxy, alkylsulphonyloxy or arylsulphonyloxy.

4. Fungicidal agents, characterised in that they contain at least one azole-substituted oximino-cyano-acetamide derivative of the general formula (I) according to Claims 1 and 3.

5. Process for combating fungi, characterised in that azole-substituted oximino-cyano-acetamide derivatives of the formula (I) according to Claims 1 and 3 are allowed to act on plants or their habitat.

6. Use of azole-substituted oximino-cyano-acetamide derivatives of the formula (I) according to Claims 1 and 3, for combating fungi.

7. Process of preparation of fungicidal agents, characterised in that azole-substituted oximino-cyano-acetamide derivatives of the formula (I) according to Claims 1 and 3 are mixed with extenders and/or surface-active agents.

**Revendications**

1. Dérivés d'oximino-cyan-acétamide azol-substitués de formule générale

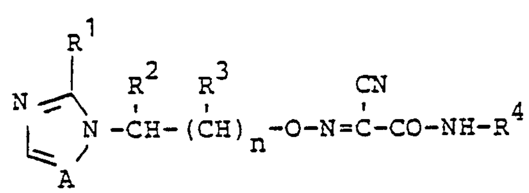

(I)

dans laquelle

$R^1$ et $R^3$ représentent de l'hydrogène et un alcoyle ayant jusqu'à 3 atomes de carbone,

$R^2$ de l'hydrogène, un alcoyle ayant jusqu'à 6 atomes de carbone et un halogénoalcoyle ayant jusqu'à 2 atomes de carbone et jusqu'à 5 atomes d'halogène,

$R^4$ de l'hydrogène et le groupe CO—NH—$R^5$,

$R^5$ signifiant de l'hydrogène, un cycloalcoyle ayant 3 à 7 et un alcoyle ayant jusqu'à 8 atomes de

carbone, ce dernier pouvant être substitué par des groupes cyano, hydroxycarbonyle, aminocarbonyle, alcoxy et alcoxycarbonyle, avec chaque fois jusqu'à 4 atomes de carbone dans la portion alcoyle,

A représente N ou le groupe CH et

n les nombres 0 ou 1.

2. Composés de formule générale (I) selon la revendication 1,

A représentant un atome d'azote ou le groupe CH,

$R^1$ et $R^3$ de l'hydrogène ou un méthyle,

$R^2$ de l'hydrogène ou un alcoyle ayant 1 à 4 atomes de carbone,

$R^4$ de l'hydrogène ou le groupe CO—CH—$R^5$,

$R^5$ signifiant de l'hydrogène, un cycloalcoyle ayant 3 à 6 atomes de carbone ou un alcoyle ayant 1 à 5 atomes de carbone dans la portion alcoyle, qui est éventuellement substitué par des groupes cyano ou alcoxy ayant jusqu'à 4 atomes de carbone, et

n étant les nombres 0 ou 1.

3. Procédé de préparation de dérivés d'oximino-cyan-acétamide azol-substitués de formule générale

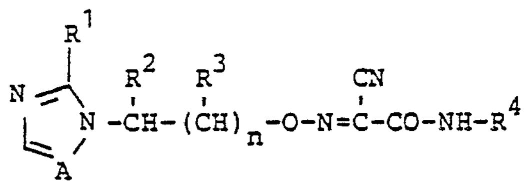

(I)

dans laquelle

$R^1$ et $R^3$ représentent de l'hydrogène et un alcoyle ayant jusqu'à 3 atomes de carbone,

$R^2$ de l'hydrogène, un alcoyle ayant jusqu'à 6 atomes de carbone et un halogénoalcoyle ayant jusqu'à 2 atomes de carbone et jusqu'à 5 atomes d'halogène,

$R^4$ de l'hydrogène et le groupe CO—NH—$R^5$,

$R^5$ représentant de l'hydrogène, un cycloalcoyle ayant 3 à 7 et un alcoyle ayant jusqu'à 8 atomes de carbone, ce dernier pouvant être substitué par des groupes cyano, hydroxycarbonyle, aminocarbonyle, alcoxy et alcoxycarbonyle et ayant chaque fois jusqu'à 4 atomes de carbone dans la portion alcoyle,

A signifiant N ou CH et

n représentant les nombres 0 ou 1,

caractérisé en ce que

a) on fait réagir un N-alcoylazol de formule

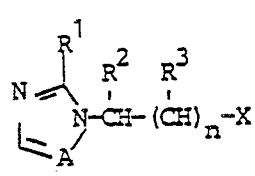

(II)

dans laquelle

$R^1$, $R^2$, $R^3$, A et N possèdent les significations indiquées plus haut et

X représente un groupe qui se détache, comme du chlore, du brome, de l'iode ou un groupe sulfonyloxy,

ou son sel, avec un dérivé de 2-oximino-2-cyan-acétamide de formule

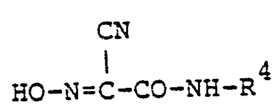

(III)

dans laquelle

$R^4$ possède la signification indiquée plus haut,

en présence d'un agent fixateur d'acide ; en outre en ce que

b) pour les dérivés d'oximino-cyano-acétamide azol-substitués de formule (I), dans lesquels $R^4$ représente le groupe CO—NH—$R^5$, $R^5$ ayant les significations citées plus haut à l'exception d'hydrogène et d'hydroxycarbonylalcoyle, on les obtient aussi lorsqu'on fait réagir les composés conformes à l'invention préparés selon le procédé a), de formule

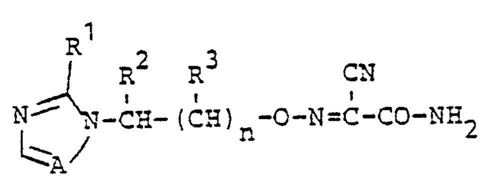

(IV)

dans laquelle

$R^1$, $R^2$, $R^3$, A et n possèdent les significations citées plus haut,

en présence d'une base forte, avec un isocyanate de formule

$$OCN—R^5 \qquad (V)$$

dans laquelle

$R^5$ possède les significations citées plus haut à l'exception d'hydrogène et d'hydroxycarbonylalcoyle, et en ce que

c) pour la préparation des composés de formule (I) dans lesquels $R^4$ représente CO—CH—$R^5$, $R^5$ prenant ici la signification alcoyl-carbonyloxyalcoyle, on fait réagir un acide carboxylique synthétisé selon le procédé a), de formule

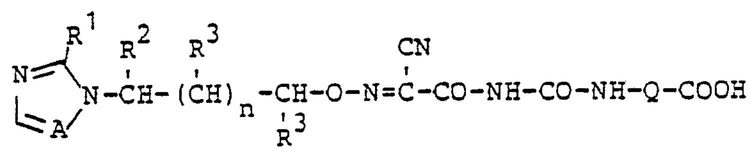

(VI)

dans laquelle

$R^1$, $R^2$, $R^3$, A et n possèdent les significations citées plus haut,

Q représentant un alcoylène à chaîne droite ou ramifiée ayant 1 à 8 atomes de carbone,

sous la forme de son sel alcalin, alcalino-terreux ou d'ammonium, avec un agent d'alcoylation de formule

$$Y—R^6 \qquad (VII)$$

dans laquelle

$R^6$ représente un alcoyle ayant 1 à 4 atomes de carbone et

Y un groupe qui se détache, comme du chlore, du brome, de l'iode, un alcoxysulfonyloxy, un alcoylsulfonyloxy ou un arylsulfonyloxy.

4. Agents fongicides, caractérisés par une teneur en au moins un dérivé d'oximino-cyan-acétamide azol-substitué de formule générale (I) selon les revendications 1 et 3.

5. Procédé pour combattre les champignons, caractérisé en ce qu'on fait agir des dérivés d'oximino-cyan-acétamide azol-substitués de formule (I) selon les revendications 1 et 3 sur des plantes ou sur leur espace vital.

6. Utilisation de dérivés d'oximino-cyan-acétamide azol-substitués de formule (I) selon les revendications 1 et 3 pour combattre les champignons.

7. Procédé de fabrication d'agents fongicides, caractérisé en ce qu'on mélange des dérivés d'oximino-cyan-acétamide azol-substitués de formule (I) selon les revendications 1 et 3 avec des diluants et/ou des agents tensioactifs.